# EUROPEAN PATENT SPECIFICATION

(11) **EP 2 515 859 B1**
(45) Date of publication and mention of the grant of the patent: **16.09.2015**
(21) Application number: 10805548.4
(22) Date of filing: 24.12.2010
(51) Int. Cl.: A61K 9/46, A61K 9/20, A61K 9/00, A61K 31/545

(54) **RAPIDLY DISPERSING EFFERVESCENT FORMULATION**
SCHNELL DISPERGIERENDE BRAUSEFORMULIERUNG
FORMULATION EFFERVESCENTE À DISPERSION RAPIDE

(30) Priority: 25.12.2009 TR 200909785; 04.05.2010 TR 201003547
(43) Date of publication of application: 31.10.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000260
(87) International publication number: WO 2011/078830

(56) References cited:
- WO-A1-2004/104010
- WO-A2-2007/129176
- DE-A1-102007 002 924
- US-A1- 2007 134 325
- DATABASE WPI Week 200630 Thomson Scientific, London, GB; AN 2006-285271 XP002620814, & CN 1 706 389 A (JINAN PINGZHI MEDICINE SCI TECH CO LTD) 14 December 2005 (2005-12-14)
- PRAKASH V. ET AL.: "Oral and parenteral therapeutic options for outpatient urinary infections caused by Enterobacteriaceae producing CTX-M extended spectrum beta-lactamases.", ANTIMICROBIAL AGENTS AND CHEMOTHERAPY, vol. 53, no. 3, 5 January 2009 (2009-01-05), pages 1278-1280, XP002628190, DOI: 10.1128/AAC.01519-08

## Description

Present invention is related to effervescent pharmaceutical dosage forms comprising cefdinir as active agent and processes for preparation of these formulations.

### Background of the invention

Cefdinir molecule which is shown with Formula I was first disclosed in the patent numbered BE897864 and its chemical name is (6R,7R)-7-[[(2Z)-(2-amino-4-thiazolil)(hydroxyimino)acetyl] amino]-3-ethenyl-8-oxo-5-thia-1-azabicyclo[4.2.0]oct-2-en-2-carboxylic acid. The molecule, which is a third generation cephalosporin, is indicated for the treatment of several illnesses caused by gram positive and gram negative bacteria.

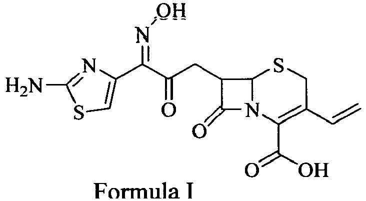

Cefdinir which physically appears as a white powder has very poor solubility in common organic solvents such as methanol, ethanol, and acetonitrile and in water. Due to this property there are some problems while developing effervescent formulations comprising this molecule and in the bioavailability of the finished product.

Depending on the formulation, some differences are seen in the physical properties of the finished product, for example some parameters such as hardness and dispersion time. Hardness of the tablets is important for carrying tablets without any breaking and using them safely; dispersion time is important for dispersing rapidly of effervescent product in water and getting the product ready for use in a short time.

As the tablet gets harder, the fragility becomes lower but this effect the dispersion and dispersion becomes slower. This situation proves that in addition to all other physical properties of the pharmaceutical formulations (for instance; flowability and solubility of the formulation etc.), tablet hardness and dispersion time should be optimized sensitively. Document CN 1706389 (published on 2005-12-14) discloses cefdinir effervescent formulation comprising polyvinylpyrrolidone and sodium hydrogen carbonate.

Considering the prior art, it is seen that new formulations are required to prepare tablets for having optimum tablet hardness and dispersion time.

The inventors have surprisingly found that the problems, which are present in the prior art, can be solved by effervescent cefdinir formulation according to the present invention.

### Detailed description of the invention

The present invention is the effervescent formulation comprising cefdinir as an active agent, characterized in that said formulation comprises polyvinylpyrrolidone in an amount in the range of 2-5% with respect to the total weight of the unit dose and trometamol which is as an organic base.

The present invention is related to effervescent tablets which disperse in water in less than 120 seconds, preferably 120 seconds and comprise tablet hardness in the range of 7-8 kP and which comprise cefdinir as an active agent. Surprisingly, it was found that in effervescent formulations comprising cefdinir as an active agent, the formulation obtained by the use of polyvinyl pyrolidone in an amount in the range of 2-5% compared to the total weight of the unit dose, dispersed in a short time, that is less than 120 seconds, in water and the tablets obtained by using this formulation had the hardness of 7-8 kP. Therefore, effervescent tablet formulations which do not break during process and carrying and also dissolve in water rapidly were developed.

Accordingly, one aspect of the present invention is effervescent tablets which disperse in water in less than 120 seconds, preferably less than 110 seconds and have a tablet hardness of 7-8 kP and comprise cefdinir as an active agent.

Another aspect of the present invention is the effervescent formulations comprising cefdinir as an active agent in which polyvinyl pyrolidone is used in an amount in the range of 2-5% compared to the total weight of the unit dose.

In the studies, it was seen that the use of polyvinyl pyrolidone plays an important role in obtaining the desired dispersion time and tablet hardness values.

Several different substances can be used as a binder in the pharmaceutical formulations. The inventors selected polyvinyl pyrrolidone from these alternatives. Polyvinyl pyrrolidone is compared with the other binders which have similar structure to that of polyvinyl pyrolidone. The results of this comparison are represented in Table 1 and Table 2. In Table 1, the dissolution times of effervescent tablet formulations prepared by binders which are very similar to polyvinyl pyrolidone are shown. In tables, there are some data obtained from the use of hydroxypropyl cellulose (HPS), microcrystalline cellulose (MCC), hydroxypropyl methyl cellulose (HPMC), polyvinyl pyrrolidone (PVP) and pregelatinized starch.

Each binder was added into the formulation in an amount of 4% of the weight of the unit dose and all the formulations are produced with the same production method. The results obtained indicates that binders providing the dissolution in a shortest time are HPS, PVP and pregelatinized starch.

**Table 1: Dissolution time of the formulation comprising different binders.**

| Binder | Dissolution time (second) |
|---|---|
| Hydroxylpropylcellulose | 123 |
| Microcrystalline cellulose | 148 |
| Hydroxypropyl methyl cellulose | 167 |
| Polyvinyl pyrrolidone | 108 |
| Pregelatinized starch | 100 |

The hardness values of the tablets formed by the effervescent formulations obtained by using different binders are illustrated in Table 2.

**Tablo 2: Hardness values of the tablets obtained by the formulations comprising different binders.**

| Binder | Tablet hardness (kP) |
|---|---|
| Hydroxylpropyl cellulose | 4.2 |
| Microcrystalline cellulose | 11.1 |
| Hydroxypropyl methyl cellulose | 13 |
| Polyvinyl pyrrolidone | 7.5 |
| Pregelatinized starch | 4 |

As it is seen from these data, the formulations, in which the dissolution occurs in a short time and tablet hardness is not low, are obtained by using PVP as a binder.

Effervescent formulation in accordance with the present invention can be stored in tablet and/or sachet forms.

Effervescent formulation in accordance with the present invention comprises cefdinir, polyvinyl pyrrolidone and in addition to these, the other pharmaceutically acceptable excipients, for instance; basic agent, effervescent acid, effervescent base, sweetener, lubricant, coloring agent and flavoring agent.

Cefdinir which can be used in effervescent formulation of the present invention can be present in the form of its solvates, hydrates, enetiomers, racemates, organic salts, inorganic salts, polymorphs, crystal and amorphous forms or in free form and/or as a combination of these. Basic agent which is used in the effervescent formulation of the present invention is trometamol,

Lubricant which can be used in the effervescent formulation according to the present invention can be selected from, but not limited with, a group comprising calcium stearate, magnesium stearate, polyethylene glycol, PEG6000, polyvinyl alcohol, potassium benzoate, sodium benzoate.

Sweetener which can be used in effervescent formulation according to the present invention, can be selected from, but not limited with, a group comprising acesulfame, aspartamate, dextrose, fructose, glucose, lactitol, maltitol, maltose, sorbitol, saccharide, sodium saccharide, sodium cyclamate, sucralose, sodium chloride, potassium chloride, sucrose, xylitol or combinations thereof.

Effervescent acid which can be used in according to the present invention, can be selected from organic acids such as citric acid, tartaric acid, malic acid, furmaric acid etc and effervescent base can be selected from basic agents such as sodium carbonate, sodium hydrogen carbonate, potassium carbonate, potassium hydrogen carbonate etc.

In effervescent formulations that will be produced by the process of the present invention, 1-4000 mg of cefdinir or its pharmaceutically acceptable salts, hydrates, solvates and/or a combination of these in an amount equivalent to that can be used.

Another aspect of the present invention is that effervescent formulation comprising cefdinir includes compared to the total weight of the unit dose;
- 5-60% of cefidinir or solvates, hydrates, enantiomers, racemates, organic salts, inorganic salts, polymorphs, crystal and amorphous forms thereof and
- 1-30% organic base, which is trometamol.
- 2-5% polyvinyl pyrrolidone
- 0.1-5% lubricant
- 0.1-5% sweetener
- 0.1-8% coloring agent and/or flavoring agent and
- 0.1-90% effervescent couple

In the effervescent cefdinir formulation in accordance with the present invention, optionally a second active agent can be used. The second active agent can be selected from beta lactamase and cephalasporins, preferably clavulanic acid or combinations thereof.

Clavulanic acid used in the effervescent cefdinir formulation in accordance with the present invention, can be present in the form of its solvates, hydrates, enetiomers, racemates, organic salts, inorganic salts, polymorphs, crystal and amorphous forms or in free form and/or as a combination of these. Preferably, potassium clavulanate is used.

In the effervescent cefdinir formulation according to the present invention, optionally 50-500 mg of clavulanic acid or pharmaceutically acceptable salts, hydrates, solvates or a combination thereof in an amount equivalent to that can be used.

Clavulanic acid and/or derivatives thereof (for example potasium clavulanate) is very sensitive to moisture. Therefore, in the pharmaceutical composition according to the present invention, potasium clavulanate is preferably used together with a desiccant in a ratio of 1:1.

One or more than one of the following substances can be used as a desiccant; silica; colloid silica, for instance colloidal silica anhydrous, Aerosil® 200, magnesium trisilicate, powder cellulose, Cabosil®, magnesium oxide, calcium silicate, Syloid®, starch, microcrystalline cellulose and talk.

In the effervescent cefdinir formulation in accordance with the present invention, potasium clavulanate is preferably used with syloid or microcrystalline cellulose in a ratio of 1:1.

In the effervescent cefdinir formulation in accordance with the present invention, compared to the total weight of the unit dose, 5-90%, preferably 10-80%, of clavulanic acid or pharmaceutically acceptable salts, solvates, hydrates or a combination thereof in an amount equivalent to that can be used.

Another aspect of the present invention is the processes for the preparation of effervescent cefdinir formulations which disperse in water in less than 120 seconds, have a tablet hardness of 7-8 kP and comprise polyvinyl pyrrolidone in an amount in the range of 2-5%.

Said processes comprise the following steps;
- Granulation of cefdinir and effervescent base by aqueous solution of basic agent
- Mixing obtained granules with sweetener and effervescent acid and granulation of them with the solution comprising polyvinyl pyrrolidone in an amount in the range of 2-5%
- Finally, mixing obtained granules with coloring agent, flavoring agent, sweetener and lubricant, and optionally filling into the sachets or pressing them in tablet forms.

Though not limited by these examples, effervescent formulations in accordance with the present invention can be prepared according to the following examples.

### EXAMPLE 1: Formulation and process for the preparation of effervescent tablet

| | % amount in unit dose |
|---|---|
| Cefdinir | 30 % |
| Organic base | 9 % |
| Citric acid | 33 % |
| Sodium hydrogen carbonate | 20 % |
| Polyvinyl pyrrolidone | 2.5 % |
| Sweetener | 2.5 % |
| Lubricant | 0.75 % |
| Coloring agent | 1.25 % |
| Flavoring Agent | 1 % |

Formulation, that will be prepared in accordance with the present invention, is obtained by granulation of sodium hydrogen carbonate and cefdinir with aqueous solution of organic base and then mixing the formed granules with citric acid and sweetener. The formed mixture is then granulated with a solution of polyvinyl pyrrolidone. The granule obtained after this step is mixed with lubricant, coloring agent, sweetener and flavouring agent and optionally it can be compressed as tablets.

### EXAMPLE 2: Formulation and process for the preparation of effervescent tablet

| | % amount in unit dose |
|---|---|
| Cefdinir | 20 % |
| Potassium clavulanate:syloid | 13 % |
| Organic base | 9 % |
| Citric acid | 30 % |
| Sodium Hydrogen Carbonate | 20 % |
| Polyvinyl pyrrolidone | 2.5 % |
| Sweetener | 2.5 % |
| Lubricant | 0.75 % |
| Coloring agent | 1.25 % |
| Flavoring Agent | 1 % |

Formulation can be obtained by granulation of cefdinir with aqueous solution of organic base and then mixing the formed granules with sweetener, effervescent acid and potassium clavulanate: syloid. The formed mixture is then granulated with a solution of binder. The granule obtained after this step is mixed with lubricant, coloring agent, sweetener and flavouring agent and optionally it can be pressed as tablets.

In another aspect present invention relates to use of effervescent formulations comprising cefdinir and in addition to that pharmaceutically acceptable excipients for the treatment of infections caused by gram positive and gram negative bacteria.

In another aspect pharmaceutical formulation prepared in accordance with the present invention, is used for the manufacture of a medicament for use in the treatment and prophylaxis of upper respiratory tract infections such that ear, nose, throat, otitis media, sinusitis, tonsillitis, pharyngitis, lower respiratory tract infections such as pyelonephritis, cystitis and urethritis, skin and soft tissue infections such as froncle, pyoderma, impetigo and also gonorrhea and lyme diseases.

## Claims

1. An effervescent formulation comprising cefdinir as an active agent, **characterized in that** said formulation comprises polyvinyl pyrrolidone in an amount in the range of 2-5% with respect to the total weight of the unit dose and trometamol which is as an organic base.

2. The effervescent formulation according to claim 1, wherein said formulation is in tablet or sachet form.

3. The effervescent formulation according to claim 2, wherein said formulation is in tablet form.

4. The effervescent formulation according to claims 1-3, wherein said formulation disperses in water in less than 120 seconds.

5. The effervescent formulation according to claims 3 and 4, wherein the hardness of the tablet form is in the range of 7-8 kP.

6. The effervescent formulation comprising cefdinir according to claim 1, wherein said compoisition comprises pharmaceutically acceptable excipients, such as; basic agent, effervescent acid, effervescent base, sweetener, lubricant, coloring agent and flavoring agent in addition to cefdinir and polyvinyl pyrolidone.

7. The effervescent formulation according to any of the previous claims, wherein cefdinir is present in the form of its solvates, hydrates, enetiomers, racemates, organic salts, inorganic salts, polymorphs, crystal and amorphous forms or in free form and/or as a combination thereof.

8. The effervescent formulation comprising cefdinir according to claim 7, wherein lubricant is selected from a group comprising calcium stearate, magnesium stearate, polyethylene glycol, PEG6000, polyvinyl alcohol, potassium benzoate, sodium benzoate.

9. The effervescent formulation comprising cefdinir according to claim 7, wherein sweetener is selected from a group comprising acesulfame, aspartamate, dextrose, fructose, glucose, lactitol, maltitol, maltose, sorbitol, saccharide, sodium saccharide, sodium cyclamate, sucralose, sodium chloride, potassium chloride, sucrose, xylitol or combinations thereof.

10. The effervescent formulation comprising cefdinir according to claim 7, wherein effervescent acid is selected from organic acids such as citric acid, tartaric acid, malic acid, furmaric acid etc and effervescent base is selected from basic agents such as sodium carbonate, sodium hydrogen carbonate, potassium carbonate, potassium hydrogen carbonate etc.

11. The effervescent cefdinir formulation prepared according to any of the previous claims, wherein said formulation comprises the followings compared to the total weight of the unit dose;
• 5-60% of cefdinir or its solvates, hydrates, enantiomers, racemates, organic salts, inorganic salts, polymorphs, crystal and amorphous forms thereof and
• 1-30% organic base, which is trometamol,
• 2-5% polyvinyl pyrrolidone
• 0.1-5% lubricant
• 0.1-5% sweetener
• 0.1-8% coloring agent and/or flavoring agent and
• 0.1-90% effervescent couple

12. The effervescent cefdinir formulation prepared according to any of the previous claims, wherein in addition to cefdinir, a second active agent selected from beta lactamase and/or cephalasporins is used.

13. A formulation according to claim 12, wherein potassium clavulanate, is used as the second active agent.

14. A process that will be used in the production of a formulation according to claim 1, wherein said process comprises the following steps;
• Granulation of cefdinir and effervescent base by aqueous solution of basic agent
• Mixing the obtained granules with sweetener and effervescent acid and granulating them with the solution comprising polyvinyl pyrrolidone in an amount in the range of 2-5%
• Finally, mixing obtained granules with coloring agent, flavoring agent, sweetener and lubricant, and optionally filling into the sachets or pressing them in tablet forms.

15. A formulation according to any of the previous claims for use in the treatment and prophylaxis of upper respiratory tract infections such that ear, nose, throat, otitis media, sinusitis, tonsillitis, pharyngitis, lower respiratory tract infections such as pyelonephritis, cystitis and urethritis, skin and soft tissue infections such as froncle, pyoderma, impetigo and also gonorrhea and lyme diseases.

## Patentansprüche

1. Eine Brauseformulierung, die Cefdinir als Wirkstoff beinhaltet, **dadurch gekennzeichnet, dass** die genannte Formulierung Polyvinylpyrrolidon in einer Menge im Bereich von 2-5%, bezogen auf das Gesamtgewicht der Einheitsdosis und Trometamol, eine organische Base, beinhaltet.

2. Die Brauseformulierung nach Anspruch 1, wobei die genannte Formulierung in Form von Tabletten oder Sachet ist.

3. Die Brauseformulierung nach Anspruch 2, wobei die genannte Formulierung in Form von Tabletten ist.

4. Die Brauseformulierung nach Ansprüchen 1-3, wobei die genannte Formulierung sich in Wasser in weniger als 120 Sekunden auflöst.

5. Die Brauseformulierung nach den Ansprüchen 3-4, wobei die Härte der Tablettenform im Bereich von 7-8 kp ist.

6. Die Brauseformulierung enthaltend Cefdinir nach Anspruch 1, wobei die genannte Zusammensetzung pharmazeutisch verträgliche Hilfsstoffe wie basische Mittel, Brausesäure, Brausebasis, Süßstoff, Gleitmittel, Färbemittel und Geschmacksmittel zusätzlich zu Cefdinir und Polyvinylpyrrolidon beinhaltet.

7. Die Brauseformulierung nach einem der vorhergehenden Ansprüche, wobei Cefdinir in Form seiner Solvate, Hydrate, Enetiomers, Racemate, organischen Salze, anorganischen Salze, Polymorphe, Kristall- und amorphen Formen oder in freier Form und / oder als Kombination davon vorhanden ist.

8. Die Brauseformulierung enthaltend Cefdinir nach Anspruch 7, wobei Gleitmittel aus einer Gruppe enthaltend Calciumstearat, Magnesiumstearat, Polyethylenglykol, PEG 6000, Polyvinylalkohol, Kaliumbenzoat und Natriumbenzoat ausgewählt wird.

9. Die Brauseformulierung enthaltend Cefdinir nach Anspruch 7, wobei Süßstoff aus einer Gruppe enthaltend Acesulfam, Aspartamate, Dextrose, Fructose, Glucose, Lactitol, Maltitol, Maltose, Sorbit, Saccharide, Natrium -Saccharid, Natrium-Cyclamat, Sucralose, Natriumchlorid, Kalium chlorid, Saccharose, Xylitol oder Kombinationen davon ausgewählt wird.

10. Die Brauseformulierung enthaltend Cefdinir nach Anspruch 7, wobei Brausesäure aus organischen Säuren wie Zitronensäure, Weinsäure, Äpfelsäure, Fumarsäure usw. ausgewählt wird und Brausebasis aus basischen Mitteln wie Natriumcarbonat, Natriumhydrogencarbonat, Kalium- Carbonat, Kaliumhydrogencarbonat usw. ausgewählt wird.

11. Die Brause Cefdinir-Formulierung, die nach einem der vorhergehenden Ansprüche vorbereitet wird, wobei die genannte Formulierung die Folgenden im Vergleich zu dem Gesamtgewicht der Einheitsdosis beinhaltet;
• 5-60% von Cefdinir
oder seiner Solvate, Hydrate, Enantiomere, Racemate, organischen Salze, anorganischen Salze, Polymorphe, Kristall- und amorphen Formen davon und
• 1-30% der organischen Base, die Trometamol ist,
• 2-5% von Polyvinylpyrrolidon
• 0.1-5% von Gleitmittel
• 0.1-5% von Süßstoff
• 0.1-8% von Färbemittel und/oder Aromamittel und
• 0.1-90% von Brausepaar

12. Die Brause Cefdinir-Formulierung, die nach einem der vorhergehenden Ansprüche vorbereitet wird, wobei neben Cefdinir ein zweiter Wirkstoff aus Beta-Lactamase und/oder Cephalasporins verwendet wird.

13. Eine Formulierung nach Anspruch 12, wobei Kaliumclavulanat als der zweite Wirkstoff verwendet wird.

14. Ein Verfahren, das in der Herstellung einer Formulierung nach Anspruch 1 umgesetzt wird, wobei das genannte Verfahren die folgenden Schritte umfasst;
• Granulierung von Cefdinir und Brausebasis durch wässrige Lösung des basischen Mittels
• Vermischen der erhaltenen Granulate mit Süßstoff und Brausesäure und Granulieren sie mit der Lösung, die Polyvinylpyrrolidon in einer Menge im Bereich von 2-5% beinhaltet
• Schließlich, Mischen die erhaltenen Granulate mit Färbemittel, Geschmacksmittel, Süßungsmittel und Gleitmittel und gegebenenfalls Einfüllen in Sachets oder Pressen sie in Tablettenformen.

15. Eine Formulierung nach einem der vorhergehenden Ansprüche zur Verwendung bei der Behandlung und Prophylaxe von Infektionen der oberen Atemwege wie Ohr, Nase, Hals, Mittelohrentzündung, Sinusitis, Tonsillitis, Pharyngitis, Infektionen der unteren Atemwege wie Pyelonephritis, Zystitis und Urethritis, Haut- und Weichteilinfektionen wie froncle, Pyodermie, Impetigo und auch Tripper und Lyme-Krankheiten.

## Revendications

1. Une formulation effervescente comprenant cefdinir comme l'agent actif **caractérisée en ce que** ladite formulation comprend la polyvinylpyrrolidone dans un montant de 2-5% par rapport au poids total de la dose unitaire et le trométamol qui est une base organique.

2. La formulation effervescente selon la revendication 1 dans laquelle ladite formulation est sous la forme d'un comprimé ou d'un sachet.

3. La formulation effervescente selon la revendication 2 dans laquelle ladite formulation est sous la forme d'un comprimé.

4. La formulation effervescente selon les revendications 1-3, dans laquelle ladite formulation se disperse dans l'eau en moins de 120 secondes.

5. La formulation effervescente selon les revendications 3 et 4, dans laquelle la dureté du comprimé est dans la plage de 7-8 kP.

6. La formulation effervescente comprenant cefdinir selon la revendication 1, dans laquelle ladite composition comprend des excipients pharmaceutiquement acceptables, tels que; l'agent basique, l'acide effervescent, la base effervescente, l'édulcorant, le lubrifiant, l'agent colorant et l'agent aromatisant en plus du cefdinir et la polyvinylpyrrolidone.

7. Une formulation effervescente selon quelconque des revendications précédentes, dans laquelle, cefdinir est présent sous la forme de ses solvates, hydrates, énantiomères, racémates, sels organiques, sels inorganiques, polymorphes, sous la forme cristalline et amorphe ou bien sous la forme libre et/ou d'une combinaison de ceux-ci.

8. La formulation effervescente comprenant cefdinir selon la revendication 7, dans laquelle le lubrifiant est choisi d'un groupe comprenant le stéarate de calcium, le stéarate de magnésium, le polyéthylèneglycol, PEG 6000, l'alcool de polyvinyle, le benzoate de potassium, le benzoate de sodium.

9. La formulation effervescente comprenant cefdinir selon la revendication 7, dans laquelle l'édulcorant est choisi d'un groupe comprenant l'acésulfame, l'aspartame, le dextrose, le fructose, le glucose, le lactilol, le maltilol, le maltose, le sorbitol, le saccharide, le saccharide de sodium, le cyclamate de sodium, le sucralose, le chlorure de sodium, le chlorure de potassium, le saccharose, le xylitol, ou les combinaisons de ceux-ci.

10. La formulation effervescente comprenant cefdinir selon la revendication 7, dans laquelle l'acide effervescente est choisi parmi les acides organiques telles que l'acide citrique, l'acide tartrique, l'acide malique, l'acide fumarique etc. et la base effervescente est choisi parmi les agents basiques tels que le carbonate de sodium, l'hydrogénocarbonate de sodium, le carbonate de potassium, l'hydrogénocarbonate de potassium, etc.

11. La formulation effervescente de cefdinir préparée selon quelconque des revendications précédentes, dans laquelle ladite formulation comprend les suivants par rapport au poids total de la dose unitaire;
• 5-60% de cefdinir
ou ses produits de solvatation, hydrates, énantiomères, racémates, sels organiques, sels inorganiques, polymorphes, formes cristallines et formes amorphes de ceux-ci et
• 1-30% de base organique étant trométamol,
• 2-5% de polyvinylpyrrolidone
• 0.1-5% de lubrifiant
• 0.1-5% d'édulcorant
• 0.1-8% d'agent colorant et/ou d'agent aromatisant et
• 0.1-90% de couple effervescent.

12. La formulation effervescente de cefdinir préparée selon quelconque des revendications précédentes, dans laquelle en plus de cefdinir, un deuxième agent actif choisi parmi les bêta-lactamases et/ou les céphalasporines est utilisé.

13. Une formulation selon la revendication 12, dans laquelle le clavulanate de potassium est utilisé comme deuxième agent actif.

14. Un procédé qui sera utilisé dans la fabrication d'une formulation selon la revendication 1, dans lequel ledit procédé comprend les étapes suivantes:
• La granulation du cefdinir et la base effervescente par une solution aqueuse de l'agent basique
• Mélanger les granules obtenus avec l'édulcorant et l'acide effervescent et les granuler avec la solution comprenant la polyvinylpyrrolidone dans un montant de 2-5%
• Finalement, mélanger des granules obtenus avec l'agent colorant, l'agent aromatisant, l'édulcorant et le lubrifiant, et éventuellement les remplir dans des sachets ou les presser sous la forme de comprimés.

15. Une formulation selon quelconque des revendications précédentes pour utilisation dans le traitement et la prophylaxie des infections des voies respiratoires supérieures tels que des oreilles, du nez, de la gorge, de l'otite moyenne, de la sinusite, de l'amygdalite, de la pharyngite, des infections des voies respiratoires inférieures telles que la pyélonéphrite, de la cystite et urétrite, des infections cutanées et des tissus mous, comme froncle, pyodermite, l'impétigo et également de la gonorrhée et de maladies de Lyme.
